# EUROPÄISCHE PATENTSCHRIFT

(11) **EP 1 459 066 B1**
(45) Veröffentlichungstag und Bekanntmachung des Hinweises auf die Patenterteilung: **14.11.2007**
(21) Anmeldenummer: 02758055.4
(22) Anmeldetag: 19.06.2002
(51) Int. Cl.: G01N 33/543

(54) **VERFAHREN ZUR MESSUNG VON MIKROVISKOSITÄTSVERÄNDERUNGEN ALS IMMUNTEST**
METHOD FOR MEASURING CHANGES IN MICROVISCOSITY SERVING AS AN IMMUNE TEST
PROCEDE DE MESURE DE VARIATIONS DE MICROVISCOSITE SERVANT DE TEST IMMUNOLOGIQUE

(30) Priorität: 21.06.2001 DE 10130727
(43) Veröffentlichungstag der Anmeldung: 22.09.2004
(73) Patentinhaber: Mediquant GmbH, 06120 Halle/S (DE)
(72) Erfinder: WILHELM, Michael, 06108 Halle (DE); AGUEEV, Vladimir, 06114 Halle (DE)
(74) Vertreter: Voigt, Wolf-Rüdiger
(86) Internationale Anmeldenummer: PCT/DE2002/002262
(87) Internationale Veröffentlichungsnummer: WO 2003/001207

(56) Entgegenhaltungen:
- EP-A- 0 287 665
- US-A- 4 725 140
- US-A- 4 762 413

## Beschreibung

Die Erfindung betrifft ein lasergestütztes Verfahren zur Messung von Mikroviskositätsveränderungen in Flüssigkeiten, wenn spezifische Reaktionen zwischen zwei Komponenten ablaufen. Im Ergebnis von Mikroviskositätsveränderungen werden mit Hilfe der Computertechnik elektrische Signale ausgewertet, so dass beispielsweise Antigen-Antikörper-Reaktionen eindeutig nachweisbar sind.

Mit der Erfindung erfolgt die Erfassung chemischer und biochemischer Reaktionen mit einer Sensivität im Sub-Pico-Bereich. Eine breite analytische und diagnostische Nutzung ist beispielsweise in den Bereichen Medizin, Veterinärmedizin, Umweltanalytik, Chemie. Biochemie oder Pharmazie möglich. Beispiele sind die bereits genannten Antigen-Antikörper-Reaktionen sowie Prionenbindungen an spezifische Komponenten in der TSE-Diagnostik, enzymkatalysierte Reaktionen, Proteinstrukturänderungen sowie Nachweis von Umweltschadstoffen oder Drogen mittels spezifisch bindender Komponenten.

Es sollen nunmehr Ausführungen zum Stande der Technik erfolgen, wobei es sich bei den bekannten Verfahren meistens um Mehrschritt-Immuntestverfahren handelt.

Die bisher beschriebenen Immuntestverfahren detektieren spezifische Antigen-Antikörper-reaktionen mit verschiedenen Markierungen, im folgenden als "Marker" bezeichnet.

Die hauptsächlich eingesetzten Marker sind radioaktive Substanzen (RIA-Radioimmunoassay), Enzyme (EIA-Enzymimmunoassay), Fluoreszenzfarbstoffe (FIA-Fluoreszenzimmunoassay) oder Mikropartikel und magnetische Mikropartikel. Diese Marker werden zur quantitativen und qualitativen Darstellung der Immunkomplexe eingesetzt.

Z.B. magnetische Partikel haben die Aufgabe, sich an Antikörper oder Antigene anzulagern. Dann können in einem Probenvolumen spezifisch reagierende Antigene bzw. Antikörper als Immunkomplexe nach Anlegung eines elektromagnetischen Feldes von den nicht gebundenen Antigenen oder Antikörpern getrennt werden. Über die Konzentration der Marker mittels sensitiver Detektionsgeräte werden die Immunkomplexe quantifiziert.

Gemäß EP 0 287 665 B1 wird ein lasermagnetisches Immuntestverfahren mit entsprechender Vorrichtung beschrieben. Entsprechend dem vorgenannten Europapatent werden magnetische Mikropartikel mit einem Reaktionspartner gebunden und sollen als Reaktionspartner für zu detektierende Antigene oder Antikörper dienen. Die Schritte diese Verfahrens sind im wesentlichen folgende:

Es erfolgt ein Markieren eines Antigens oder Antikörpers mit magnetischen Mikropartikeln, das Unterwerfen der erhaltenen magnetischen Substanz einer Probe zu einer möglichen Antigen-Antikörper-Reaktion, das Leiten des erhaltenen markierten Immunkomplexes in eine Meßposition nach der Entfernung von unbenutzter markierter Substanz und optische Messung der Konzentration der magnetischen Mikropartikel als Marker der Immunkomplexe. Unter Hinweis auf Seite 7 der vorgenannten Europäischen Patentschrift umfaßt die Erfindung folgende Schritte:

Ausbildung eines magnetischen Körper-Proben-Komplexes durch immunologisches Umsetzen einer Probe mit einem magnetisch markierten Körper, Entfernen des unbenutzten magnetisch markierten Körper, Abtrennen und Dispergieren des Komplexes, Leiten und Konvertieren des Komplexes mit magnetischen Mitteln und Messen der Proben durch Bestrahlen des Komplexes mit einem Laser und Nachweis der austretenden Lichteintensität, worin ein periodisch wechselndes Magnetfeld auf den Komplex angelegt wird, der in einem ausgewählten Ort durch magnetische Kraft konzentriert ist und Nachweis von nur der austretenden Lichtintensität, die in Synchronisation mit der Frequenz des periodisch wechselnden Magnetfeldes ist. Es wird die Intensität des Streulichtes des Laserstrahls gemessen, das proportional der Konzentration der magnetischen Mikropartikel ist.

Die Nachweisempfindlichkeit des Verfahrens beträgt 10⁻¹² g.

Es handelt sich bei dem EP 0 287 665, wie oben schon gesagt, um ein Mehrschritt-Verfahren. Diese Erfindung schreibt zwingend vor, dass markierte Immunkomplexe separiert werden müssen. Anderenfalls wäre eine Quantifizierung der Immunkomplexe nicht gegeben. Die magnetischen Partikel haben also die Aufgabe der Markierung, Konzentrierung der Reaktionsprodukte und/oder Separierung der spezifisch gebundenen Partikel.

Bei dem o. g. Europapatent zeigen sich folgende Nachteile:

Es liegt eine Mehr-Schritt-Methode vor mit der nicht im Minutenbereich gemessen werden kann. Die Zeit zur Herstellung des Markerkomplexes muss der Messzeit für Immunkomplexe zugerechnet werden. So dauert beispielsweise die Gesamtzeit zur Detektion der Immunkomplexe mehr als 1 Stunde.

Aufgrund der Messdauer kann die Dynamik der Immunreaktionen nicht dargestellt werden, da nur das Endergebnis nach Inkubationszeit erfasst werden kann. Die Methode stellt also keine Echtzeit-Messmethode dar.

Es sei zu den Mehrschritt-Verfahren noch angemerkt, daß die Protokolle zur Vermeidung falscher Ergebnisse äußerst genau abgearbeitet werden müssen. Waschung, Separierung. Markierung, konstante Temperatur und pH-Werte können bei geringen Abweichungen zu Fehlern führen. Jeder kleine Fehler der Teilschritte addiert sich zu einem grossen im Endergebnis. So kann beispielsweise während der Separierung eine Konvektionsströmung oder Sedimentation auftreten und zu fehlerhaften Messungen führen.

Da nur Teilvolumina der Probe mit dem Laserstrahl gemessen werden, ergeben sich Fehlermöglichkeiten durch ungenaue Konzentrierung der mikropartikel-markierten Immunkomplexe.

Nach der Separierung der magnetischen Mikropartikel mit den Immunkomplexen wird mit dem Laserstrahl die Intensität der Oszillationen des Streulichtes gemessen, wobei kritisch anzumerken ist, daß die Intensität der Oszillationen nicht nur von der Konzentration der magnetischen Mikropartikel abhängt.

Zu den bekannten Mehrschritt-Verfahren kann gesagt werden, dass oft Mikropartikel genutzt werden, die sich durch ihre Eigenschaften unterscheiden. Diese Mikropartikel werden meistens zur Markierung beispielsweise von Antigenen und Antikörpern oder Immunkomplexen genutzt.

Charakteristisch für das Markieren ist, dass Partikel oder magnetische Partikel mit hoher Affinität zu Antigenen oder Antikörpern ausgewählt werden und die Basis für das Funktionieren der Immuntestverfahren darstellen. Zum Beispiel werden magnetische Mikropartikel mit organischen Polymeren verkapselt, die wiederum spezifisch mit Antigen oder Antikörpern binden sollen.

Es bestehen auch Ein-Schritt-Methoden als Immuntest wie z.B. Patent US 4762413. In diesem Patent wird die Immunreaktion über das Fourierspektrum der Bewegungen der Mikropartikel durch Braun'sche Molekularbewegung vor und nach Reaktion gemessen. Zur Messung der Fourierspektra wird das Speckle-Interferometer eingesetzt. Der Hauptnachteil dieser Methode ist, dass kleinste Vibrationen überlagernde Störgeräusche verursachen, da keine Synchron- oder Korrelationsmessung angewendet wird.

US-Patent US4725140 beschreibt ein Verfahren, bei dem magnetische Mikropartikel, die einen Antikörper tragen, in einem Immuntest eingesetzt werden.

Genannt seien ferner das Europapatent 0 287 665 B1 und US-Patent 4762413, die der noch im einzelnen darzulegenden Erfindung am nächsten kommen.

Es ist die Aufgabe der Erfindung, unter Berücksichtigung von Mikroviskositätsveränderungen ein lasergestütztes Immuntestverfahren vorzuschlagen, welches als schnelles Echtzeit-Einschritt-Verfahren es möglich macht, die Nachweisempfindlichkeit spezifischer Reaktionen und/oder Immunkomplexe gegenüber dem Stand der Technik zu erhöhen. Mit dem neuen Verfahren soll eine Nachweisempfindlichkeit im sub-pico-Bereich möglich sein. Durch die hohe Sensivität des Verfahrens soll eine frühzeitige Erkennung u.a. von Viruserkrankungen. Krebserkrankungen, Amyloid-Erkrankungen, TSE-Erkrankungen und nekrotisierende Erkrankungen ermöglicht werden.

Erfindungsgemäß wird die Aufgabe wie folgt gelöst, wobei hinsichtlich der grundlegenden erfinderischen Gedanken auf den Patentanspruch 1 verwiesen wird. Die weitere Ausgestaltung der Erfindung ergibt sich aus den Patentansprüchen 2 bis 11.

Es soll im Hinblick auf die weiteren Erläuterungen zur Erfindung noch einmal klar herausgestellt werden, dass durch Messung von Mikroviskositätsveränderungen als Folge von spezifischen Reaktionen zwischen zwei Komponenten (z. B. Antigen-Antikörper-Reaktionen) ein neues hochsensitives Verfahren als Immuntest vorgeschlagen wird. Die im Ergebnis von spezifischen Reaktionen zwischen zwei Komponenten auftretenden Mikroviskositätsveränderungen führen eindeutig zu reproduzierbaren Ergebnissen. Oder anders ausgedrückt: Zur Lösung der oben formulierten Aufgabe wird die Erfassung der veränderlichen Mikroviskosität einer flüssigen Probe als Ergebnis einer Antigen-Antikörper-Reaktion oder anderer biochemischer Reaktionen genutzt.

Die Veränderung der Mikroviskosität ist abhängig von der räumlichen Größenzunahme der Moleküle bzw. der Immunkomplexe oder der Ausbildung dreidimensionaler Immunkomplexnetze.

Detektierbare Veränderungen der Mikroviskosität hängen von der Konzentration der spezifisch bindenden Komponenten ab. In Mikroküvetten kann die Konzentration der Komponenten erhöht werden. In Mehr-Schritt-Messverfahren unterliegen die Protokolle einem definierten Konzentrations-Verhältnis zwischen den Komponenten. Das hier beschriebene Verfahren kann mit dramatisch unterschiedlichen oder zufälligen Konzentrationsverhältnissen der Komponenten messen.

In einem transparenten Probegefäß, z.B einer Mikroküvette, befindet sich als Flüssigkeit eine Testkomponente, die als Reaktionskomponente im Hinblick auf den weiteren Verfahrensablauf anzusehen ist. Der Mikroküvette werden inerte magnetische Mikropartikel zugegeben. Diese Probe mit den magnetischen Mikropartikeln wird einem gepulsten Elektromagnetfeld ausgesetzt, um die Veränderungen der Partikelbeweglichkeit erfassen zu können. Für eine bessere Beweglichkeit (Schwingung, Drehung, Geschwindigkeit. Beschleunigung u.s.w.) der magnetischen Mikropartikel im Magnetfeld können die Partikel magnetisiert werden. Während der Messung ist die Mikropartikelkonzentration konstant, die Mikropartikel befinden sich in der Küvette in einem homogenen Zustand, da die Anordnung der Probe mit Mikropartikeln in einem zu jedem Zeitpunkt homogenen, gepulsten Magnetfeld erfolgt, das zu Bewegungen der Mikropartikel führt, ohne die Homogenität und Konzentration der Mikropartikel zu verändern. Hervorzuheben ist, dass sich die Probe in einem homogenen gepulsten Elektromagnetfeld befindet, d. h., dass alle Mikropartikel sich in einem Äquipotenzial-Magnetfeld befinden, also in jeder Zeit an jedem Ort homogene Verhältnisse bestehen. Im Gegensatz zum vorgenannten EP sind die Magnetkerne wesentlich grösser als die Grösse der Mikroküvette. Diese Situation führt zu dem genannten homogenen Elektromagnetfeld.

Um einen Diffusierungsprozeß der Mikropartikel zum Elektromagneten zu verhindern, werden beispielsweise zwei Elektromagneten in gegenüberliegender Position verwendet mit Anordnung der Mikroküvette im Zentrum. Durch periodischen Spannungswechsel von einem Elektromagneten zum anderen wird eine gerichtete Bewegung der magnetischen Mikropartikel verhindert.

Hier ist im Hinblick auf den anfangs erläuterten Stand der Technik als bedeutsam herauszustellen, daß die magnetischen Mikropartikel in keinem Fall die Funktion von Markern erfüllen, sondern als Probenkörper für Mikroviskositätsmessungen dienen, die aus den Änderungen der Bewegungen der magnetischen Mikropartikel im gepulsten, homogenen Elektromagnetfeld resultieren. Für einen verbesserten Reaktionsprozess zwischen den Komponenten wird zusätzlich der Mixeffekt der beweglichen magnetischen Mikropartikel genutzt.

Nunmehr folgt die Zugabe einer zweiten Reaktionskomponente, wodurch es gegebenenfalls zu spezifischen Reaktionen zwischen den Komponenten kommt. Es ist jetzt möglich, die durch spezifische Reaktionen eintretende Veränderung der Mikroviskosität der flüssigen Probe ohne Trennung der Reaktionsbestandteile von den übrigen Bestandteilen zu messen. Binden sich die Antikörper mit spezifischen Antigen, erfolgt ein Anstieg der Mikroviskosität. Bei unspezifischen Antigenen und Antikörpern wird die Mikroviskosität nicht ansteigen.

Die detektierten Zeitveränderungen, die aus Veränderungen der Geschwindigkeit oder Beschleunigung der magnetischen Partikel resultieren, sind proportional zur Mikroviskosität der flüssigen Probe, unter den Vorgaben des gepulsten, stabilisierten Elektomagnetfeldes, konstanter Temperatur und homogener Mikropartikelgrösse. In dieser Situation ist bedeutsam, dass die Messergebnisse absolut unabhängig von der Konzentration der magnetischen Mikropartikel sind.

Die Mikroviskosität kann nicht nur über die detektierten Zeitveränderungen, sondern auch über die Amplituden der Schwingung und Drehung gemessen werden unter der Voraussetzung, dass die Konzentration der magnetischen Mikropartikel im untersuchten Probenvolumen und die Amplitude des oszillierenden Magnetfeldes konstant sind.

Die elektrischen Signale werden folgendermaßen ermittelt. Ein stabilisierter Laserstrahl durchdringt die Mikroküvette vertikal. Dabei beleuchtet der Laserstrahl das gesamte Probenvolumen. Diese Situation führt zu einer Unterscheidung vom o.g. EP 0 287 665 B1 dadurch, dass Sedimentation die Konzentration der magnetischen Mikropartikel nicht verändert, da die Anzahl der magnetischen Mikropartikel in der gesamten Küvette gleich bleibt.

Zur Messung der Amplituden der Schwingungen und Drehungen oder Geschwindigkeit und Beschleunigung wird ein Speckle-Interferometer benutzt. Das Streulicht (nach Austritt aus der Mikroküvette) zeigt das Speckle-Muster, das als Ergebnis der Interferenz des Streulichts, das von den Mikropartikeln ausgeht, anzusehen ist. Über Fotodetektoren werden die Intensitätsfluktuationen der Speckle-Muster in unterschiedlichen Messpunkten erfasst, diese elektrischen Signale werden einem Differenzialverstärker zur weiteren Verarbeitung zugeführt, danach übernimmt ein A/D-Konverter die Signalweiterleitung zur mathematischen Kalkulation in einem Computer. Die hier beschriebene Erfindung nutzt eine Korrelationsmessung der Intersitätsfluktuationen der Speckle-Muster um ein besseres Signal/Geräusch-Verhältnis zu erhalten. Die Intensitätsfluktuationen werden mit 2 oder mehr Fotodetektoren gemessen, deren Anordnung die Störgeräusche (z.B. mechanische Geräusche oder Lasergeräusche) korreliert, die nützlichen Signale jedoch nicht korreliert. Diese Korrelationsdistanz wird durch die Formel k= (λ·L) : (π·r) bestimmt. Dabei ist k die Korrelationsdistanz der Speckle-Muster, λ ist die Wellenlänge des Laserlichts, L ist die Entfernung zwischen dem Zentrum der Messküvette und der Detektionsebene und r ist der Durchmesser des Laserstrahls in der Messküvette. Der Abstand zwischen den Fotodetektoren d muss grösser als die Korrelationsdistanz k sein. Nach der Subtraktion der Signale im Differenzialverstärker mit hoher Genauigkeit werden die korrelierten Störgeräusche dramatisch reduziert (> 90 dB), während das nicht-korrelierte nützliche Signal verstärkt wird. Diese Korrelationsmessung wird sowohl im Einzelimpuls-Magnetfeld als auch im oszillierenden Magnetfeld durchgeführt. Bei Nutzung des oszillierenden Magnetfeldes wird zusätzlich das nützliche Signal in Synchronisation mit der Frequenz dieses Magnetfeldes erfasst.

Die Erfindung soll nunmehr anhand eines Ausführungsbeispiels erläutert werden.

Die Figuren stellen dar:
- Figur 1:: Schematische Darstellung der Vorrichtung zur Messung von Mikroviskositätsveränderungen
- Figur 2:: Das Oszillogramm der Intensitätsfluktuationen der Speckle-Muster im Einzelimpuls-Magnetfeld mit niedriger Mikroviskosität
- Figur 3:: Das Oszillogramm der Intensitätsfluktuationen der Speckle-Muster im Einzelimpuls-Magnetfeld bei hoher Mikroviskosität
- Figur 4:: Mikroviskositätsveränderungen während einer Antigen-Antikörper-Reaktion in der Zeit
- Figur 5:: Fourierspektrum der Intensitätsfluktuationen von Speckle-Mustern im oszillierenden Magnetfeld mit einer Fundamentalfrequenz 20 Hz
- Figur 6:: Die grafische Darstellung einer Antigen-Antikörper-Reaktion mit der Amplitude der fundamentalen Frequenz in der Zeit

Die einzelnen Positionszeichen bedeuten:
1 - Bauggruppe zum Aussenden stabilisierter Laserstrahlung
2 - Baugruppe zum Aussenden elektrischer Impulse
3 - Elektromagneten zum Erzeugen homogener gepulster elektromagnetischer Felder
4 - lichtdurchlässige Küvette
5 - Fotodetektoren
6 - Differentialverstärker
7 - A/D-Konverter
8 - Computer-Auswerteeinheit
D1, D2-Dioden
L - Entfernung zwischen Zentrum der Messküvette und Ebene der Fotodetektoren
d - Abstand der Fotodetektoren
µ - Winkel zwischen dem Anstieg des Magnetfeldimpulses und dem Anstieg der Kurve des Oszillogramms der magnetischen Mikropartikel
V - Volt
M - relative Mikroviskosität
t - Zeit
l - Intensität in relativen Einheiten
f - Frequenz in Hertz
A - relative Amplitude der Fundamentalfrequenz
B - relative Amplitude der 2. harmonischen Frequenz
C - relative Amplitude der 3. harmonischen Frequenz

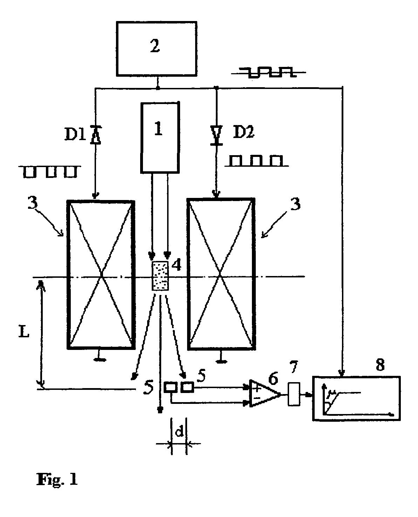

Gemäß Figur 1 werden von einer Baugruppe 1 stabilisierte Laserstrahlen ausgesendet, die auf die lichtdurchlässige Küvette 4 treffen. In der Küvette befindet sich die zu untersuchende Probe. In der Küvette 4 befinden sich in einer Flüssigkeit spezifisch bindende Substanzen und inerte magnetische Mikro-Partikel. Unter der Verwendung der Baugruppe 2 werden elektrische Impulse mit einem Kurvenverlauf erzeugt, wie er beispielsweise in Figur 1 neben der Baugruppe 2 dargestellt ist. Diese Impulse werden mit Hilfe der Dioden D1 und D2 in positive und negative Impulse umgewandelt (Darstellung der Impulse in Figur 1 nahe der Dioden D1 und D2). Die beiderseits der Küvette 4 angeordneten Elektromagneten 3 erzeugen in der Küvette ein gepulstes homogenes Magnetfeld um die magnetischen Mikropartikel zu bewegen. Die aus der Küvette 4 austretende Strahlung trifft auf die Fotodetektoren 5. Mit Hilfe eines Differenzverstärkers 6 werden die in den Fotodetektoren 5 gewonnenen elektrischen Signale subtrahiert und die Differenz verstärkt und über einen A/D-Konverter 7 einer Auswerteinheit 8 zugeführt, die die Mikroviskosität darstellt und aufzeichnet.

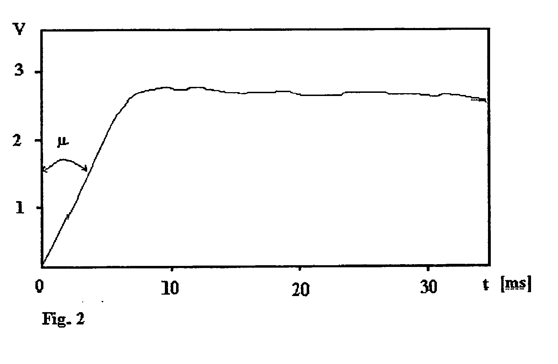

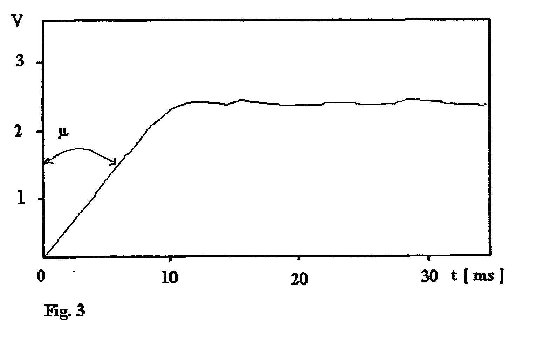

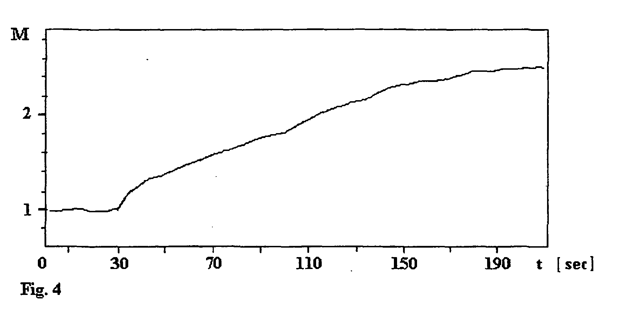

Der bis hierher beschriebene Verfahrensablauf ergibt sich anschaulich aus Figur 2. Hier sieht man das Oszillogramm der Intensitätsfluktuationen der Speckle-Muster im Einzelimpuls-Magnetfeld mit niedriger Mikroviskosität. Bei t=0 beginnt der Anstieg des Magnetfeld-Einzelimpulses. Das Oszillogramm zeigt die Bewegungen der magnetischen Mikropartikel. Dabei zeigt der Winkel µ zwischen dem Anstieg des Magnetfeldimpulses und dem Anstieg der Kurve des Oszillogramms der magnetischen Mikropartikel die Mikroviskosität der Probe. In Figur 3 sieht man die Veränderung des Winkels µ als Resultat einer höheren Viskosität, wenn die zweite spezifisch bindende Komponente der Probe zugeführt wurde. In der Figur 4 sieht man die Veränderung der Mikroviskosität in der Zeit. Zum Zeitpunkt t = 30 sec. wurde die zweite spezifisch bindende Komponente zugegeben. Dabei wird positive Antwort des Systems innerhalb von Sekunden gegeben. Die Konzentration der Immunkomplexe in dieser Messung lag im sub-pico-Bereich. Durch die Messung der Mikroviskosität vor und nach Zugabe der spezifisch bindenden Komponenten zeigt die Differenz die Veränderung der Mikroviskosität, die nicht sensitiv für die Temperatur ist. Zur Messung der Dynamik in der Echtzeitmessung muss jedoch die Temperatur der Probe stabilisiert sein.

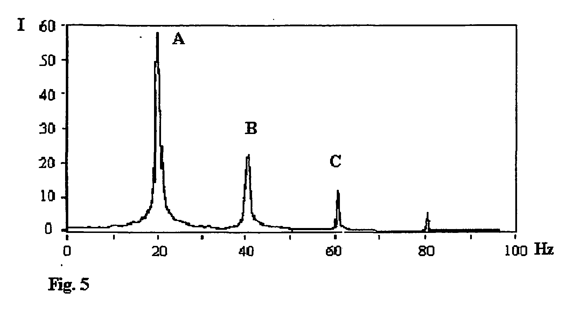

Figur 5 zeigt das Fourier-Spekrum der Intensitätsfluktuationen von Speckle-Mustern im oszillierenden Magnetfeld mit einer Fundamentalfrequenz 20 Hz. Dies ist eine alternative Messung zur Geschwindigkeits- und Beschleunigungsmessung. Die Amplituden des Fourier-Spektrums sind umgekehrt proportional zur Mikroviskosität. Die Amplituden sind direkt proportional zur Konzentration der magnetischen Mikropartikel, deshalb wird zur fehlerfreien Detektion des Probenvolumens eine während der Messung konstante Konzentration der magnetischen Mikropartikel benutzt. Die konstante Konzentration wird durch das oben beschriebene homogene Magnetfeld und die vertikale Anordnung des Laserstrahls mit vollständiger Beleuchtung des Probenvolumens erreicht. Die Differenzialmessung der Änderungen der Mikroviskosität kann nicht nur mit der Amplitude der Fundamentalfrequenz sondern auch mit der zweiten oder höheren harmonischen Frequenzen gemessen werden.

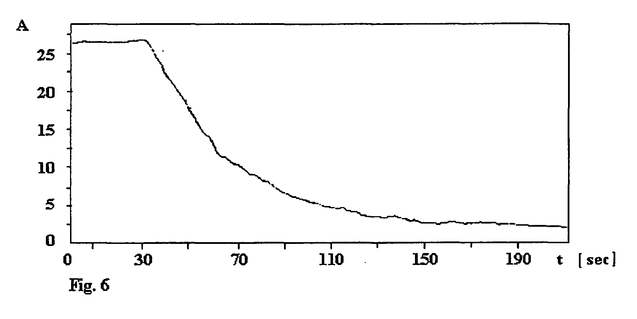

Figur 6 zeigt die grafische Darstellung einer Antigen-Antikörper-Reaktion mit der Amplitude der fundamentalen Frequenz in der Zeit. Zum Zeitpunkt t = 30 sec. wurde die zweite spezifisch bindende Komponente zugegeben. Im Gegensatz zur Figur 4 ist die Amplitude der fundamentalen Frequenz oder der harmonischen Frequenzen umgekehrt proportional zur Mikroviskosität, deshalb sieht man in Figur 6 eine abfallende Kurve bei Viskositätszunahme.

Schlußfolgernd ist festzustellen:

Das Echtzeit-Ein-Schritt-Verfahren bedarf demnach nicht der Trennung der Reaktionsprodukte von den übrigen Bestandteilen einer flüssigen Probe , wie dies bei den Mehrschritt-Testverfahren in den Testdurchführungsprotokollen beschrieben wird. Nach diesen Protokollen werden in verschiedenen Stadien der Testabläufe Trennungsschritte (z.B. mit Magnetpartikeln oder Gelelektrophorese) und/oder Waschschritte durchgeführt. Erst nach Trennung und Anreicherung der Immunkomplexe im Magnetfeld wird die Qualifizierungsmessung durchgeführt. Damit sind die Mehrschritt-Immunoassays nicht in der Lage dynamische und zeitnahe Reaktionsabläufe darzustellen. Die Kinetik der Antigen-Antikörper-Reaktionen verschließt sich dem Mehrschritt-Immuntest-Anwender.

In unserem Verfahren werden in einem homogenen, gepulsten Magnetfeld die Veränderungen der Mikroviskosität als Ergebnis der spezifischen Reaktion zwischen zwei Reaktionspartnern in einem Arbeitsschritt gemessen. Die magnetischen Mikropartikel sind für die in dem flüssigen Probenvolumen eingesetzten Substanzen inert. Am Immunkomplex sind die Partikel nicht beteiligt.

Gegenüber dem Stande der Technik, wobei nochmals auf das EP 0 287 665 B1 verwiesen wird, ergeben sich mit dem erfindungsgemäßen Verfahren und der entsprechenden Vorrichtung folgende Vorteile bzw. Abweichungen:
- Es wird ein Einschritt-Verfahren vorgeschlagen
- In dieser Methode werden keine Marker verwendet
- Es werden Mikroviskositätsveränderungen gemessen (und nicht die Konzentration der Mikropartikel)
- Die inerten magnetischen Partikel werden als Testkörper zur Detektion der Mikroviskosität verwendet
- Es wird das gesamte Mikrovolumen der Probe gemessen
- In der Meßküvette herrscht ein homogenes, gepulstes Magnetfeld
- Die Konzentration der magnetischen Partikel ist während der Messung in der Mikroküvette konstant
- Es wird zur Detektion der Intensität der Fluktuationen der Speckle-Muster die Korrelationsmethode eingesetzt.

## Patentansprüche

1. Verfahren zur Messung von Mikroviskositätsveränderungen als Immuntest, wobei eine flüssige Probe, die sich in einer lichtdurchlässigen Mikroküvette (4) befindet, eine spezifische Reaktionskomponente wie Antigene oder Antikörper enthält, die möglicherweise zur Bildung von Immunkomplexen führen und weiterhin inerte magnetische Mikropartikel enthält und diese Reaktionskomponente wegen der inerten Eigenschaft der magnetischen Mikropartikel keine Bindung mit den inerten magnetischen Mikropartikeln eingeht, die Probe mit einem stabilisierten Laser bestrahlt wird und eine stabilisierte Temperatur aufweist, der Inhalt der Mikroküvette (4) einem homogenen, stabilisierten gepulstem Magnetfeld ausgesetzt wird, so dass mit Hilfe des gepulsten Magnetfelds die inerten magnetischen Mikropartikel bewegt werden, registrierbare Intensitätsfluktuationen der Speckle-Muster in elektrische Signale umgewandelt werden, die eindeutig der Mikroviskosität zugeordnet werden können und nachdem dem Küvetteninhalt eine zweite Reaktionskomponente zugegeben wurde, im Fall der Bildung von Immunkomplexen die ansteigende Mikroviskosität durch Veränderung der Geschwindigkeit oder der Beschleunigung der inerten magnetischen Mikropartikel zur Qualifizierung und Quantifizierung der Immunkomplexe genutzt wird.

2. Verfahren nach Anspruch 1, **dadurch gekennzeichnet, dass** die inerten magnetischen Mikropartikel einen bestimmten Größebereich und Konzentration haben.

3. Verfahren nach Anspruch 1, **dadurch gekennzeichnet, dass** der Laserstrahl die Mikroküvette vertikal durchtritt und das gesamte Probenvolumen in der Küvette ausleuchtet.

4. Verfahren nach Anspruch 1, **dadurch gekennzeichnet, dass** der Vektor der Stärke des gepulsten Magnetfeldes senkrecht zur optischen Achse des Laserstrahls ausgerichtet wird.

5. Verfahren nach Anspruch 1, **dadurch gekennzeichnet, dass** die Mikroviskosität von der Messung der Geschwindigkeit der magnetischen Mikropartikel nach Applizierung von Einzelimpulsen des Magnetfeldes bestimmt wird.

6. Verfahren nach Anspruch 1 und 5, **dadurch gekennzeichnet, dass** die Mikroviskosität von der Messung der Beschleunigung der magnetischen Mikropartikel nach Applizierung von Einzelimpulsen des Magnetfeldes bestimmt wird.

7. Verfahren nach Anspruch 1, **dadurch gekennzeichnet, dass** die Mikroviskosität über die Messung der Amplituden der Peaks des Fourier-Spektrums der Intensitätsfluktuationen von Speckle-Mustern bestimmt wird, wenn die Probe im stabilisierten oszillierenden Magnetfeld einer konstanten Fundamentalfrequenz ausgesetzt ist.

8. Verfahren nach Anspruch 1, **dadurch gekennzeichnet, dass** die magnetischen Mikropartikel vorhergehend magnetisiert werden.

9. Verfahren nach Anspruch 1, **dadurch gekennzeichnet, dass** unter Verwendung der Dioden D 1 und D 2 die Elektromagneten (3) wechselweise mit Impulsen angeregt werden.

10. Verfahren nach Anspruch 1, **dadurch gekennzeichnet, dass** die Intensitätsfluktuationen der Speckle-Muster mit 2 oder mehr Fotodetektoren gemessen werden, die einer Entfernung d angeordnet werden, die größer als k = (λ L) : (π r) ist, so dass die Störgeräusche korreliert, die nützlichen Signale jedoch nicht korreliert werden und danach die elektrischen Signale der Fotodetektoren zu verschiedenen Eingängen eines Differenzialverstärkers geleitet werden.

11. Verfahren nach Anspruch 1 und 10, **dadurch gekennzeichnet, dass** die Intensitätsfluktuationen der Speckle-Muster in Synchronisation mit dem gepulsten Magnetfeld gemessen werden.

## Claims

1. Method of measuring changes in microviscosity serving as an immune test, whereby a liquid sample located in a transparent microcuvette (4) contains a specific reaction constituent such as antigens or antibodies, which may lead to the formation of immune complexes and in addition contains inert magnetic microparticles and this reaction constituent does not bond with the inert magnetic microparticles because of the inert property of the said magnetic microparticles, the sample is irradiated with a stabilised laser and has a stabilised temperature, the contents of the microcuvette (4) are exposed to a homogeneous, stabilised and pulsed magnetic field, so that the inert magnetic microparticles are moved with the aid of the pulsed magnetic field, registerable intensity fluctuations of the speckle patterns are converted into electrical signals, which can be clearly associated with the microviscosity and a second reaction constituent is subsequently added to the contents of the microcuvette, in the event of the formation of immune complexes the increasing microviscosity is used for qualifying and quantising the immune complexes by means of the change of velocity or acceleration of the inert magnetic microparticles.

2. Method according to claim 1 **characterised in that** the inert magnetic microparticles have a specific size range and concentration.

3. Method according to claim 1 **characterised in that** the laser beam passes vertically through the microcuvette and illuminates the entire volume of the sample in the cuvette.

4. Method according to claim 1 **characterised in that** the vector of the strength of the pulsed magnetic field is aligned vertically to the optical axis of the laser beam.

5. Method according to claim 1 **characterised in that** the microviscosity is determined by measuring the velocity of the magnetic microparticles after the application of individual impulses of the magnetic field.

6. Method according to claims 1 and 5 **characterised in that** the microviscosity is determined by measuring the acceleration of the magnetic microparticles after the application of individual impulses of the magnetic field.

7. Method according to claim 1 **characterised in that** the microviscosity is determined by the measurement of the amplitudes of the peaks of the Fourier spectrum of the intensity fluctuations of speckle patterns when the sample is exposed to a constant fundamental frequency in a stabilised oscillating magnetic field.

8. Method according to claim 1 **characterised in that** the magnetic microparticles are magnetised in advance.

9. Method according to claim 1 **characterised in that** the electromagnets (3) are alternately stimulated by impulses using the diodes D1 and D2.

10. Method according to claim 1 **characterised in that** the intensity fluctuations of the speckle patterns are measured by two or more photo detectors, which are positioned at a distance d which is greater than k = (λ, L): (π r), so that the intrinsic noises are correlated, but the useful signals are not correlated and subsequently the electrical signals of the photo detectors are conducted to different inputs of a differential amplifier.

11. Method according to claims 1 and 10 **characterised in that** the intensity fluctuations of the speckle patterns are measured in synchronisation with the pulsed magnetic field.

## Revendications

1. Procédé pour la mesure de modifications de la microviscosité comme test d'immunité, par lequel un échantillon liquide, se trouvant dans une microcuvette (4) translucide, contenant un réactif spécifique tel que des antigènes ou des anticorps, qui mènent éventuellement à la formation d'immunocomplexes, et contenant de plus des microparticules magnétiques inertes et ces réactifs n'entrant pas en liaison avec les microparticules magnétiques inertes en raison de la propriété inerte des microparticules magnétiques, l'échantillon étant balayé par un laser stabilisé et présentant une température stabilisée, le contenu de la microcuvette (4) étant exposé à un champ magnétique pulsé homogène stabilisé, si bien qu'à l'aide du champ magnétique pulsé les microparticules magnétiques inertes sont mises en mouvement, les fluctuations d'intensité enregistrables du chatoiement sont transformées en signaux électriques qui peuvent être de manière univoque attribués à la microviscosité et après qu'il fut donné un deuxième réactif au contenu de la cuvette, dans le cas de la formation d'immunocomplexes, la microviscosité ascendante étant utilisée par la modification de la vitesse ou de l'accélération à la qualification et quantification des immunocomplexes.

2. Procédé d'après la revendication 1, **caractérisé en ce que** les microparticules magnétiques inertes ont une certaine plage de dimensions et concentration.

3. Procédé d'après la revendication 1, **caractérisé en ce que** le rayon laser traverse la microcuvette verticalement et éclaire le volume total de l'échantillon dans la cuvette.

4. Procédé d'après la revendication 1, **caractérisé en ce que** le vecteur de la force du champ magnétique pulsé est dirigé verticalement par rapport à l'axe optique du rayon laser.

5. Procédé d'après la revendication 1, **caractérisé en ce que** la microviscosité est déterminée par la mesure de la vitesse des microparticules magnétiques après l'application d'impulsions simples du champ magnétique.

6. Procédé d'après les revendications 1 et 5, **caractérisé en ce que** la microviscosité est déterminée par la mesure de l'accélération des microparticules magnétiques après l'application d'impulsions simples du champ magnétique.

7. Procédé d'après la revendication 1, **caractérisé en ce que** la microviscosité est déterminée par la mesure des amplitudes des pics du spectre de Fourier des fluctuations d'intensité du chatoiement, quand l'échantillon est exposé à une fréquence fondamentale constante dans le champ magnétique oscillant stabilisé.

8. Procédé d'après la revendication 1, **caractérisé en ce que** les microparticules magnétique ont été magnétisées auparavant.

9. Procédé d'après la revendication 1, **caractérisé en ce qu'**en utilisant les diodes D 1 et D 2 les électroaimants (3) ont été excités réciproquement par des impulsions.

10. Procédé d'après la revendication 1, **caractérisé en ce que** les fluctuations d'intensité du chatoiement ont été mesurées avec 2 photo-détecteurs ou plus, qui ont été disposés à une distance d plus grande que k = (λ L) : (π r), si bien que le brouillage est mis en corrélation, mais non pas les signaux utiles et qu'après les signaux électriques des photo-détecteurs sont dirigés vers différentes entrées d'un amplificateur différentiel.

11. Procédé d'après les revendications 1 et 10, **caractérisé en ce que** les fluctuations d'intensité du chatoiement sont mesurées en synchronisation avec le champ magnétique pulsé.
